# EUROPÄISCHE PATENTSCHRIFT

(11) **EP 0 925 368 B1**
(45) Veröffentlichungstag und Bekanntmachung des Hinweises auf die Patenterteilung: **23.01.2002**
(21) Anmeldenummer: 98909424.8
(22) Anmeldetag: 09.02.1998
(51) Int. Cl.: C12P 17/18, C07D 487/04

(54) **VERFAHREN ZUR HERSTELLUNG VON ENANTIOMERENREINEN ALKOHOLEN**
METHOD FOR PRODUCING ALCOHOLS FREE OF ENANTIOMERS
MODE DE PRODUCTION D'ALCOOLS EXEMPTS D'ENANTIOMERES

(30) Priorität: 21.02.1997 DE 19707008
(43) Veröffentlichungstag der Anmeldung: 30.06.1999
(73) Patentinhaber: BASF AKTIENGESELLSCHAFT, 67056 Ludwigshafen (DE)
(72) Erfinder: DINGLER, Christoph, D-69207 Sandhausen (DE); HOLMAN, Nicholas, John, Nottingham NG4 4EW (GB)
(86) Internationale Anmeldenummer: EP9800706
(87) Internationale Veröffentlichungsnummer: WO9837221

(56) Entgegenhaltungen:
- WO-A-95/10521

## Beschreibung

Die Erfindung betrifft ein Verfahren zur Herstellung von enantiomerenreinen Alkoholen.

In einer Vielzahl von Publikationen und Patenten werden Reduktionsreaktionen mit Mikroorganismen oder Enzymen beschrieben. Nur wenige Arbeiten sind zur Reduktion von Ketonen mit heteroaromatischen Resten und speziell mit heteroaromatischen Resten in α-Position zur Carbonylgruppe erschienen.

So wird beispielsweise von Davis et al. (Appl. Environ. Microbiol., Vol. 48, No. 2, 1984: 327 - 331) die mikrobielle Reduktion von Pentoxifyllin (3,7-Dihydro-3,7-dimethyl-1-(5-oxohexyl)-1H-purin-2,6-dion) - einem Keton mit einem Heterocyclus in γ-Stellung zur Carbonylgruppe - zum entsprechenden Alkohol (3,7-Dihydro-3,7-dimethyl-1-(5-Hydroxyhexyl)-1H-purin-2,6-dion) beschrieben. Für die Reduktion mit wachsenden Mikroorganismen sind eine Kohlenstoff-, Stickstoff- und Phosphorquelle erforderlich. Über die Enantiomerenreinheit des entstehenden Alkohols wird keine Aussage gemacht.

Von Imuta et al. (J. Org. Chem. Vol. 43, No. 18, 1978: 3530 - 3532) wird ebenfalls die Synthese Pyridylethanol aus den entsprechenden Ketonen mit wachsenden Cryptococcus macerans-Kulturen in mäßigen Ausbeuten und Enantiomerenreinheiten beschrieben. Auch hier sind für die Reduktion eine Kohlenstoff- und Stickstoffquelle erforderlich.

Takeshita et al. beschreibt in Heterocycles 1987, Vol. 26, No. 12, 3051 - 3054 die Reduktion von Acetylpyridinen mit Saccharomyces cerevisiae zu Pyridylethanol in schlechten Ausbeuten.

Eine optimale mikrobielle Reduktion von Ketonen sollte vorteilhafterweise eine Reihe von Bedingungen erfüllen, wie beispielsweise:
1. hohe Enantiomerenreinheit
2. hohe chemische Ausbeute
3. hohe Selektivität des Enzyms oder Mikroorganismus
4. geringe Katalysatormengen (Enzym- oder Mikroorganismusmengen)
5. gute Löslichkeit von Edukt und Produkt unter Reaktionsbedingungen
6. gute Raum-Zeit-Ausbeute
7. leichte Reinigung der Syntheseprodukte
8. kostengünstige Synthese

In WO 95/10521 wird die chemische Synthese von 1,2,4-Triazolo(1,5-a)pyrimidinen sowie deren Verwendung in pharmazeutischen Zubereitungen beansprucht.

Aufgabe der vorliegenden Erfindung war es, eine stereoselektive Synthese zu Zwischenprodukten von 1,2,4-Triazolo(1,5-a)pyrimidinen zu entwickeln, die diese Verbindungen vorteilhafterweise mit hohen optischen Reinheiten und guten chemischen Ausbeuten liefert und die eine leichte Aufarbeitung der Produkte ermöglicht.

Diese Aufgabe wurde durch ein Verfahren zur Herstellung von enantiomerenreinen Alkoholen der Formel I (Ia oder Ib) 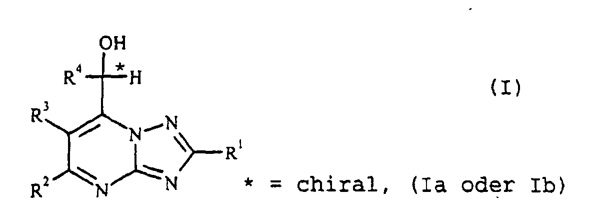 in der die Substituenten folgende Bedeutung haben:
R1
   Wasserstoff oder substituiertes oder unsubstituiertes C₁-C₆-Alkyl-, C₁-C₆-Alkoxy- oder C₁-C₆-Alkanoyl-,
R² und R³
   unabhängig voneinander Wasserstoff oder substituiertes oder unsubstituiertes C₁-C₆-Alkyl-, C₁-C₆-Alkoxy-, C₁-C₆-Alkanoyl-, C₁-C₆-Alkylthio-, C₁-C₆-Alkylsulphinyl- oder C₁-C₆-Alkylsulphonyl-,
R⁴
   R⁴ substituiertes oder unsubstituiertes C₁-C₆-Alkyl- , C₃-C₈-Cycloalkyl-,
   dadurch gekennzeichnet, daß man Verbindungen der Formel II, in der die Substituenten R¹ bis R⁴ die oben genannten Bedeutungen haben, in wäßriger Lösung 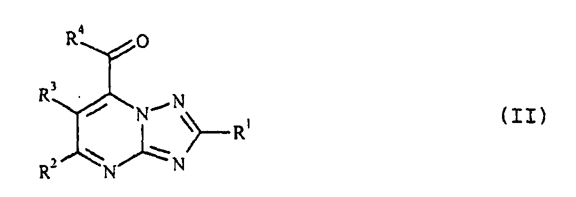 in Gegenwart einer Kohlenstoffquelle und eines Mikroorganismus oder eines Reduktionsmittels und eines Enzyms zu Verbindungen der Formel I reduziert, gelöst.

R¹ bezeichnet in den Formeln I und II Wasserstoff oder substituiertes oder unsubstituiertes C₁-C₆-Alkyl-, C₁-C₆-Alkoxy- oder C₁-C₆-Alkanoyl-

Die für R¹ genannten Reste haben beispielsweise folgende Bedeutung:
- Alkyl verzweigte oder unverzweigte C₁-C₆-Alkylketten, wie beispielsweise Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, l-Ethyl-1-methylpropyl oder 1-Ethyl-2-methylpropyl,
- Alkoxy verzweigte oder unverzweigte C₁-C₆-Alkoxyketten wie vorstehend genannt z.B, Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy,
- Alkanoyl verzweigte oder unverzweigte C₁-C₆-Alkanoylketten wie Methanoyl, Ethanoyl, Propanoyl, 1-Methylethanoyl, Butanoyl, 1-Methylpropanoyl, 2-Methylpropanoyl, 1,1-Dimethylethanoyl, Pentanoyl, 1-Methylbutanoyl, 2-Methylbutanoyl, 3-Methylbutanoyl, 1,1-Dimethylpropanoyl, 1,2-Dimethylpropanoyl, 2,2-Dimethylpropanoyl, 1-Ethylpropanoyl, Hexanoyl, 1-Methylpentanoyl, 1,2-Methylpentanoyl, 3-Methylpentanoyl, 4-Methylpentanoyl, 1,1-Dimethylbutanoyl, 1,2-Dimethylbutanoyl, 1,3-Dimethylbutanoyl, 2,2-Dimethylbutanoyl, 2,3-Dimethylbutanoyl, 3,3-Dimethylbutanoyl, 1-Ethylbutanoyl, 2-Ethylbutanoyl, 1,1,2-Trimethylpropanoyl, 1,2,2-Trimethylpropanoyl, 1-Ethyl-1-methylpropanoyl und 1-Ethyl-2-methylpropanoyl,

Als Substituenten der für R¹ genannten Reste Alkyl, Alkoxy oder Alkanoyl kommen ggf. ein oder mehrere Substituenten wie Halogen wie Fluor, Chlor, Brom, Cyano, Nitro, Amino, Thio, Alkyl, Alkoxy oder Aryl in Frage.

R² und R³ bezeichnen in den Formeln I und II unabhängig voneinander Wasserstoff oder substituiertes oder unsubstituiertes C₁-C₆-Alkyl-, C₁-C₆-Alkoxy-, C₁-C₆-Alkanoyl-, C₁-C₆-Alkylthio-, C₁-C₆-Alkylsulphinyl- oder C₁-C₆-Alkylsulphonyl-

Die für R² und R³ genannten Reste haben beispielsweise folgende Bedeutung:
- Alkyl verzweigte oder unverzweigte C₁-C₆-Alkylketten, wie beispielsweise Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl,
- Alkoxy verzweigte oder unverzweigte C₁-C₆-Alkoxyketten wie vorstehend genannt z.B, Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy,
- Alkanoyl verzweigte oder unverzweigte C₁-C₆-Alkanoylketten wie Methanoyl, Ethanoyl, Propanoyl, 1-Methylethanoyl, Butanoyl, 1-Methylpropanoyl, 2-Methylpropanoyl, 1,1-Dimethylethanoyl, Pentanoyl, 1-Methylbutanoyl, 2-Methylbutanoyl, 3-Methylbutanoyl, 1,1-Dimethylpropanoyl, 1,2-Dimethylpropanoyl, 2,2-Dimethylpropanoyl, 1-Ethylpropanoyl, Hexanoyl, 1-Methylpentanoy, 1,2-Methylpentanoyl, 3-Methylpentanoyl, 4-Methylpentanoyl, 1,1-Dimethylbutanoyl, 1,2-Dimethylbutanoyl, 1,3-Dimethylbutanoyl, 2,2-Dimethylbutanoyl, 2,3-Dimethylbutanoyl, 3,3-Dimethylbutanoyl, 1-Ethylbutanoyl, 2-Ethylbutanoyl, 1,1,2-Trimethylpropanoyl, 1,2,2-Trimethylpropanoyl, 1-Ethyl-1-methylpropanoyl und 1-Ethyl-2-methylpropanoyl,
- Alkylthio verzweigte oder unverzweigte C₁-C₆-Alkylthioketten wie Methylthio, Ethylthio, n-Propylthio, 1-Methylethylthio, n-Butylthio, 1-Methylpropylthio, 2-Methylpropylthio, 1,1-Dimethylethylthio, n-Pentylthio, 1-Methylbutylthio, 2-Methylbutylthio, 3-Methylbutylthio, 2,2-Dimethylpropylthio, 1-Ethylpropylthio, n-Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio,
- Alkylsulphinyl verzweigte oder unverzweigte C₁-C₆-Alkylsulphinylketten wie Methylsulphinyl, Ethylsulphinyl, n-Propylsulphinyl, 1-Methylethylsulphinyl, n-Butylsulphinyl, 1-Methylpropylsulphinyl, 2-Methylpropylsulphinyl, 1,1-Dimethylethylsulphinyl, n-Pentylsulphinyl, 1-Methylbutylsulphinyl, 2-Methylbutylsulphinyl, 3-Methylbutylsulphinyl, 1,1-Dimethylpropylsulphinyl, 1,2-Dimethylpropylsulphinyl, 2,2-Dimethylpropylsulphinyl, 1-Ethylpropylsulphinyl, n-Hexylsulphinyl, 1-Methylpentylsulphinyl, 2-Methylpentylsulphinyl, 3-Methylpentylsulphinyl, 4-Methylpentylsulphinyl, 1,1-Dimethylbutylsulphinyl, 1,2-Dimethylbutylsulphinyl, 1,3-Dimethylbutylsulphinyl, 2,2-Dimethylbutylsulphinyl, 2,3-Dimethylbutylsulphinyl, 3,3-Dimethylbutylsulphinyl, 1-Ethylbutylsulphinyl, 2-Ethylbutylsulphinyl, 1,1,2-Trimethylpropylsulphinyl, 1,2,2-Trimethylpropylsulphinyl, 1-Ethyl-1-methylpropylsulphinyl und 1-Ethyl-2-methylpropylsulphinyl,
- Alkylsulphonyl verzweigte oder unverzweigte C₁-C₆-Alkylsulphonylketten wie Methylsulphonyl, Ethylsulphonyl, n-Propylsulphonyl, 1-Methylethylsulphonyl, n-Butylsulphonyl, 1-Methylpropylsulphonyl, 2-Methylpropylsulphonyl, 1,1-Dimethylethylsulphonyl, n-Pentylsulphonyl, 1-Methylbutylsulphonyl, 2-Methylbutylsulphonyl, 3-Methylbutylsulphonyl, 1,1-Dimethylpropylsulphonyl, 1,2-Dimethylpropylsulphonyl, 2,2-Dimethylpropylsulphonyl, 1-Ethylpropylsulphonyl, n-Hexylsulphonyl, 1-Methylpentylsulphonyl, 2-Methylpentylsulphonyl, 3-Methylpentylsulphonyl, 4-Methylpentylsulphonyl, 1,1-Dimethylbutylsulphonyl, 1,2-Dimethylbutylsulphonyl, 1,3-Dimethylbutylsulphonyl, 2,2-Dimethylbutylsulphonyl, 2,3-Dimethylbutylsulphonyl, 3,3-Dimethylbutylsulphonyl, 1-Ethylbutylsulphonyl, 2-Ethylbutylsulphonyl, 1,1,2-Trimethylpropylsulphonyl, 1,2,2-Trimethylpropylsulphonyl, 1-Ethyl-l-methylpropylsulphonyl und 1-Ethyl-2-methylpropylsulphonyl.

Als Substituenten der für R² und R³ genannten Reste Alkyl, Alkoxy, Alkanoyl, Alkylthio, Alkylsulphinyl oder Alkylsulphonyl kommen ggf. ein oder mehrere Substituenten wie Halogen wie Fluor, Chlor, Brom, Cyano, Nitro, Amino, Thio, Alkyl, Alkoxy oder Aryl in Frage. Bevorzugt werden Fluor, Chlor, Brom, Cyano, Nitro, Amino, Thio oder Alkyl.

R⁴ bezeichnet in den Formeln I und II substituiertes oder unsubstituiertes C₁-C₆-Alkyl- oder C₃-C₈-Cycloalkyl-.

Die für R⁴ genannten Reste haben beispielsweise folgende Bedeutung:
- Alkyl verzweigte oder unverzweigte C₁-C₆-Alkylketten, wie beispielsweise Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl,
- Cycloalkyl verzweigte oder unverzweigte C₃-C₈-Cycloalkylketten mit 3 bis 8 Kohlenstoffatomen im Ring, die ggf. weitere Heteroatome im Ring oder in der Alkylkette wie N, O oder S enthalten können, wie beispielsweise Cyclopropyl, Cyclobutyl, Cyclopentyl, Cyclohexyl, Cycloheptyl, Cyclooctyl, 2-Cyclopropylpentan, 5-Cyclopropylpentan, 2-Cyclobutylbutan, 2,3-Dimethyl-3-Cyclopropylpropan oder 1-Methyl-2-Cyclopropylbutan,

Als Substituenten der für R⁴ genannten Reste Alkyl oder Cycloalkyl kommen ggf. ein oder mehrere Substituenten wie Halogen wie Fluor, Chlor, Brom, Cyano, Nitro, Amino, Thio, Alkyl, Alkoxy oder Aryl in Frage. Bevorzugt werden Fluor, Chlor, Brom, Cyano, Nitro, Amino, Thio oder Alkyl.

Für das erfindungsgemäße Verfahren (siehe Schema I) sind prinzipiell alle Mikroorganismen wie Pilze, Hefen oder Bakterien oder Enzyme oder Enzymsysteme wie die verschiedenen Alkohol- und Aldehyddehydrogenasen, die Lactat- oder Formiatdehydrogenasen bevorzugt Alkohol- und Aldehyddehydrogenasen geeignet, die Carbonylverbindungen oder Aldehyde zu den Alkoholen reduzieren können. Die Mikroorganismen können direkt nach Anzucht (Feuchtbiomasse) oder aber nach Lyophilisierung (Trockenmasse) für das erfindungsgemäße Verfahren verwendet werden. Vorteilhafterweise werden Mikroorganismen oder Enzyme verwendet, die Verbindungen der Formel I mit einer Enaniomerenreinheit von größer 85 %ee, bevorzugt größer 90 %ee und ganz besonders bevorzugt größer 95 %ee zu den entsprechenden Alkoholen reduzieren können. Als Mikroorganismen kommen beispielsweise Organismen der Gattungen Alcaligenes, Aspergillus, Beauveria, Candida, Cryptococcus, Curvularia, Diplodia, Endomycopsis, Geotrichum, Hansenula, Kloeckera, Kluveromyces, Lactobacillus, Mucor, Nocardia, Penicillium, Phaffia, Pichia, Pseudomonas , Rhodococcus, Rhodotorula, Saccharomyces, Schizosaccharomyces, Sporidiobolus, Streptomyces, Torulopsis oder Yarrowia in Frage. Vorteilhafterweise werden folgende Arten der oben genannten Gattungen verwendet: Alcaligenes eutrophus, Aspergillus niger, Aspergillus fumigatus, Beauveria bassiana, Candida guilliermondii, Candida lipolytica, Candida membranaefaciens, Candida methylica, Candida parapsilosis, Candida magnoliae, Candida rugosa, Candida utilis, Curvularia falcata, Diplodia gossypina, Cryptococcus macerans, Geotrichum candidum, Hansenula anomala, Hansenula beckii, Hansenula holstii, wingei, Hansenula polymorpha, Mucor sp., Nocardia rubropertincta, Pfaffia rhodozyma, Pichia glucozyma, Pichia fermentans, Pichia capsulata, Pichia guilliermondii, Pichia membranaefaciens, Pichia pastoris, Pseudomonas fluorescens, Pseudomonas cepacia, Rhodococcus erythropolis, Rhodococcus ruber, Rhodotorula rubra, Rhodotorula gracilis, Rhodotorula glutinis, Rhodotorulas minuta, Rhodotorula termusruber, Saccharomyces cervisiae, Saccharomyces uvarum, Saccharomyces dairensis, Saccharomyces rouxii, Saccharomyces pastorianus, Saccharomyces kluyveri, Schizosaccharomyces japonicus, Schizosaccharomyces malidevorans, Schizosaccharomyces octosporus, Schizosaccharomyces pombe, Torulopsis enokii, Torulopsis methanothermo oder Yarrowia lipolytica. Bevorzugt werden die verschiedenen Hefegattungen wie Candida, Hansenula, Kloeckera, Kluveromyces, Phaffia, Pichia, Rhodotorula, Saccharomyces, Schizosaccharomyces, Torulopsis oder Yarrowia verwendet. Besonders bevorzugt werden die Gattungen und Arten Candida guilliermondii, Candida lipolytica, Candida membranaefaciens, Candida methylica, Candida parapsilosis, Candida magnoliae, Candida rugosa, Candida utilis, Hansenula anomala, Hansenula beckii, Hansenula holstii, wingei, Hansenula polymorpha, Pfaffia rhodozyma, Pichia glucozyma, Pichia fermentans, Pichia capsulata, Pichia guilliermondii, Pichia membranaefaciens, Pichia pastoris, Rhodotorula rubra, Rhodotorula gracilis, Rhodotorula glutinis, Rhodotorulas minuta, Rhodotorula termusruber, Saccharomyces cervisiae, Saccharomyces uvarum, Saccharomyces dairensis, Saccharomyces kluyveri, Saccharomyces rouxii, Saccharomyces pastorianus, Saccharomyces kluyveri, Schizosaccharomyces japonicus, Schizosaccharomyces malidevorans, Schizosaccharomyces octosporus, Schizosaccharomyces pombe, Torulopsis enokii, Torulopsis methanothermo oder Yarrowia lipolytica, ganz besonders bevorzugt die Gattungen und Arten Saccharomyces cervisiae, Saccharomyces uvarum, Schizosaccharomyces japonicus, Pichia fermentans, Hansenula polymorpha, Rhodotorula gracilis, Candida utilis oder Candida magnoliae verwendet.

Für das erfindungsgemäße Verfahren werden die Mikroorganismen vorteilhafterweise zunächst unter stickstofflimitierten Bedingungen angezogen und nach Zellernte beispielsweise durch Zentrifugation für das erfindungsgemäße Verfahren verwendet. Die Reduktion kann mit ganzen Zellen, Zellaufschlüssen oder aus den Zellen gewonnenen Rohenzymextrakten oder gereinigten Enzymen durchgeführt werden. Das Verfahren wird vorteilhafterweise in wässrigem Medium in Gegenwart einer Kohlenstoffquelle im Falle ganzer Zellen oder eines Reduktionsmittels wie NADH oder NADPH und eines Cofaktorrecycling wie mit Hilfe der Formiatdehydrogenase und Ameisensäure und gegebenenfalls weiteren Enzymen im Falle von Zellaufschlüssen, Rohextrakten oder reinen Enzymen durchgeführt. Der Zusatz weiterer Nährstoffe bei der Reduktion mit ganzen Zellen wie eine Stickstoffquelle, Vitamine oder Phosphate ist unzweckmäßig, da unter diesen Bedingungen beispielsweise unerwünschte Nebenreaktionen beobachtet werden, die zu einer mangelnden Produktqualität oder aber zu Aufarbeitungsproblemen führen können. Als Kohlenstoffquelle für die Mikroorganismen sind alle Kohlenstoffquellen geeignet, die den Zellen die für die Reduktion notwendigen Reduktionsäquivalente liefern können. Als Kohlenstoffquellen seien hier beispielhaft Mono- oder Disaccharide wie Glucose, Mannose, Maltose, Sucrose, primäre oder sekundäre Alkohole wie Methanol, Ethanol, Propanol, Polyole wie Glycerin, niedere Carbonsäuren wie Milchsäure, Äpfelsäure oder Aminosäuren wie Glutamat genannt. Pro Gramm gebildenten Alkohol (Formel I) werden im Falle von Glucose ca. 10 g Kohlenstoffquelle benötigt.

Die Umsetzung des Edukts mit Mikroorganismen in wässriger Lösung in Gegenwart einer Kohlenstoffquelle hat den Vorteil, daß weder Edukt noch Produkt verstoffwechselt werden und keine Nebenprodukte gebildet werden.

Die Reaktion kann ohne Zugabe weiterer Lösungsmittel oder Lösungsmittelgemische in reinem Wasser oder in wässrigen Puffern durchgeführt werden. Zur Verbesserung der Löslichkeit des Edukts (Formel II) können mit wassermischbare organische Lösungmittel wie THF, Acetonitril, DMF, DMSO, Dimethylacetamid, primäre oder sekundäre Alkohole, Carbonsäuren, Lactone wie γ-Butyrolacton, die in der Lage sind die Löslichkeit des Edukts zu verbessern, der Reaktion zugesetzt werden.

Die Reaktion wird vorteilhafterweise bei einer Temperatur zwischen 0 °C und 50 °C durchgeführt, bevorzugt zwischen 10 °C und 45 °C, besonders bevorzugt zwischen 15 °C und 40 °C.

Die Reaktionszeiten betragen je nach Substrat, Mikroorganismus oder Enzym zwischen 1 bis 72 Stunden, bevorzugt zwischen 1 und 48 Stunden. Die Raum-Zeit-Ausbeute in der Reaktion liegt in einem Bereich zwischen 5 bis 150 g/l/d, vorzugsweise zwischen 30 bis 100 g/l/d, dies entspricht einer Produktbildungsrate von 0,5 bis 5 g/pro Gramm Hefe/d. Die Reaktionsausbeuten des isolierten Produkts (I) liegen in einem Bereich von 50 bis 100 %, bei einem Umsatz von 50 bis 100 %, vorzugsweise von 80 bis 100 %. Die Produktkonzentration am Ende der Reaktion liegt dabei in einem Bereich von mindestens 1 bis 40 g/l, bevorzugt zwischen 5 bis 30 g/l.

Je nach Mikroorganismus oder Enzym wird das eine oder andere Enantiomer (S-Alkohol = Formel Ia oder R-Alkohol = Formel Ib) des Produkts (Formel I) gebildet.

Der Reaktionsverlauf läßt sich leicht mit üblichen Methoden beispielsweise mittels Gaschromatographie nach Extraktion des Produkts mit einem organischen Lösungsmittel wie Essigester verfolgen.

Die Reaktion wird vorzugsweise unter aeroben Bedingungen, daß heißt im Falle der Umsetzung mit Mikroorganismen unter Belüftung, bevorzugt unter geringer Belüftung durchgeführt. Eine Umsetzung unter anearoben Bedingungen ist jedoch auch möglich.

Vorteilhafterweise sollte die Eduktkonzentration bei 50 bis 100 % der maximalen Löslichkeit des Edukts in der Lösung gehalten werden, da bei zu geringen Eduktkonzentrationen die Reaktion sehr langsam vonstatten geht und bei sehr hohen Konzentrationen die Reaktion gehemmt wird. Dies ist beispielsweise für die in Schema I genannte Verbindung der Formel 1 in einem Bereich zwischen 0,5 bis 20 g/l. Für eine rasche Reduktion sollte das Edukt in Portionen der Reaktion zugeführt werden, so daß eine Konzentration an Edukt von 30 g/l nicht überschritten wird. Das Verfahren kann kontinuierlich oder diskontinuierlich durchgeführt werden.

### Beispiele

Die Reduktion von S-(-)-7-(1-Oxoethyl)-1,2,4-triazolo[1,5-a]-pyrimidin (1) zu S-(-)-7-(1-Hydroxyethyl)-1,2,4-triazolo[1,5-a]-pyrimidin (2) mit verschiedenen Mikroorganismen (siehe Schema I) wird, wenn nicht anders beschrieben, in Wasser unter aeroben Bedingungen in Gegenwart von Glucose als einziger Kohlenstoffquelle bei Raumtemperatur (23 °C) durchgeführt. S-(-)-7-(1-Hydroxyethyl)-1,2,4-triazolo[1,5-a]-pyrimidin (2) ist als Vorprodukt für eine enantioselektive Synthese des Breitband-Antiepileptikum BTS 72664 geeignet.

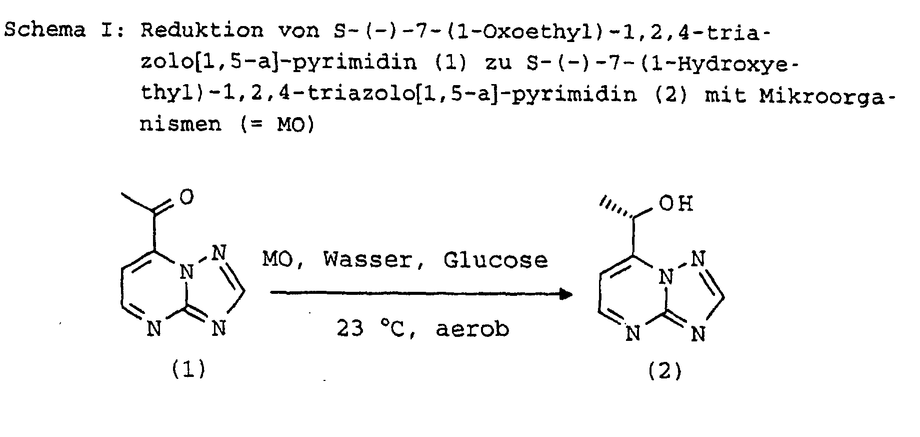

Falls nicht anders beschrieben wurde die Konzentration des Edukts und Produkts per Gaschromatographie bestimmt. Dazu wurden je 250 µl der Reaktionsbrühe als Probe mit 750 µl Essigester extrahiert und die Essigester-Phase abgenommen und per GC analysiert (GC-Säule: Methylsilicon (HP 1) 12,5 m, Injektion: 200 °C, Ofentemperatur: 50 °C für 3 min, 20 °C/min Zunahme bis auf 250 °C, 250 °C für 5 min, Injektortemperatur: 250 °C, Injektionsvolumen: 1 µl, Detektion: FID, Integration ab 1 min, Retentionszeiten: Edukt:7,95 min, Produkt: 8,73 min).

### Beispiel 1: Test verschiedener Hefestämme

Die verschiedenen in Tabelle I genannten Hefen wurden in einem bezüglich des N-Gehalts reduziertem Medium (2,5 g/l Pepton, 2,5 g/l Hefeextrakt, 20 g/l Glucose) bis zum Erreichen der stationären Phase gezüchtet, abzentrifugiert und in dem Reaktionsmedium (4 g/l Edukt (II), 75 g/l Glucose, 50 g/l Biofeuchtmasse der Heffen, 23 °C) aufgenommen. Die Reaktion wurde unter Schütteln (130 upm) für 48 h inkubiert. Nach 24 h wurden nochmals 40 g/l Glucose nachgegeben.

**Tabelle I:**

| Umsetzung mit verschiedenen Hefestämmen | | | | |
|---|---|---|---|---|
| Organismus | Edukt (g/l | Produkt (g/l) | Umsatz (%) | ee (%) |
| Saccharomyces cervisiae (Preßhefe) | 0.3 | 2.4 | 89.8 | 98.8 |
| Saccharomyces uvarum CSB 1508 | 0.6 | 1.9 | 75.6 | 98.1 |
| Saccharomyces kluyveri ATCC 22513 | 1.1 | 1.5 | 57.1 | 97.5 |
| Schizosaccharomyces japonicus DSM 70570 | 0.3 | 2.4 | 88.7 | 96.9 |
| Schizosaccharomyces pombe ATCC 38394 | 0.0 | 2.0 | 98.8 | 89.1 |
| Pichia glucozyma ATCC 18938 | 0.1 | 2.2 | 96.6 | 89.6 |
| Pichia fermentans CSB 187 | 0.3 | 2.2 | 88.0 | 99.5 |
| Hansenula polymorpha | 0.4 | 2.1 | 83.9 | 98.2 |
| Rhodotorula gracilis NRRL Y 1091 | 0.0 | 2.9 | 100.0 | 99.7 |
| Candida utilis IFO 0396 | 0.3 | 2.3 | 88.5 | 96.8 |
| Candida magnoliae ATCC 12573 | 0.1 | 2.6 | 97.7 | 98.8 |

### Beispiel 2: Reaktionskinetik

Es wurde eine Reaktionskinetik mit Presshefe der Deutschen Hefewerke in wässrigem Medium (2 g/l Edukt, 100 g/l Glucose, 50 g/l Hefe) ermittelt. Die Inkubationsbedingungen waren Bedingung A: 130 upm; 150 ml Medium in 250 ml Erlenmeyerkolben mit zwei Schikanen und Bedingung B: 140 upm; 50 ml Medium in 250 ml Erlenmeyerkolben. Beide Ansätze wurden bei 23 °C inkubiert.

**Tabelle II:**

| Reaktionskinetik mit Presshefe | | | | |
|---|---|---|---|---|
| | Bedingung A | | Bedingung B | |
| Zeit | Edukt | Produkt | Edukt | Produkt |
| (h) | (g/l) | (g/l) | (g/l) | (g/l) |
| 0h | 2.0 | 0.0 | 2.0 | 0.0 |
| 1h | 1.8 | 0.2 | 1.9 | 0.2 |
| 2h | 1.7 | 0.3 | 1.8 | 0.3 |
| 3h | 1.6 | 0.5 | 1.5 | 0.6 |
| 4h | 1.4 | 0.7 | 1.4 | 0.7 |
| 5h | 1.2 | 0.9 | 1.2 | 0.8 |
| 6h | 1.0 | 1.0 | 1.0 | 1.0 |
| 7h | 0.9 | 1.1 | 0.9 | 1.1 |
| 8h | 0.7 | 1.2 | 0.8 | 1.3 |
| 23h | 0.1 | 1.9 | 0.1 | 1.9 |

### Beispiel 3: Einfluß der Eduktkonzentration

50 g/l Presshefe (Deutsche Hefewerke) wurden in einem Standardansatz (siehe oben) mit 100 g/l Glucose bei 130 Upm inkubiert. Nach 15 h wurden nochmals 100 g/l Glucose (siehe Tabelle III, 2, 4 und 8 g/l Edukt) oder 50 g/l Glucose (siehe Tabelle III, 10 g/l Glucose) zugegeben.

**Tabelle III:**

| Einfluß der Eduktkonzentration (Deutsche Hefewerk) | | | |
|---|---|---|---|
| Edukt vorgelegt (g/l) | Zeit (h) | Edukt (g/l) | Produkt (g/l) |
| 2 | 14 | 0.39 | 1.76 |
| | 23 | 0.31 | 1.77 |
| | 38 | 0.11 | 2.03 |
| 4 | 14 | 1.01 | 3.18 |
| | 23 | 0.72 | 3.40 |
| | 38 | 0.24 | 4.01 |
| 8 | 14 | 2.30 | 5.52 |
| | 23 | 1.50 | 6.43 |
| | 38 | 4.48 | 7.77 |
| 10 | 14 | 4.88 | 4.47 |
| | 23 | 2.47 | 6.82 |
| | 38 | 0.59 | 8.94 |

### Beispiel 4: Einfluß der Eduktkonzentation

Ein Standardansatz mit 50 g/l Hefe (Fermipan, Trockenhefe der Firma Gist-Brocades) und 50 g/l Glucose wurde mit verschiedenen Eduktkonzentrationen (siehe Schema I, Verbindung 1) bei 130 Upm getestet (siehe Tabelle IV). Nach 24 h wurden nochmals 40 g/l Glucose zum. Reaktionsansatz gegeben. Oberhalb von 20 bis 30 g/l Edukt nimmt die Aktivität der Hefen deutlich ab.

**Tabelle IV:**

| Einfluß der Eduktkonzentration (50 g/l Fermipan) | | | |
|---|---|---|---|
| Keton vorgelegt (g/l) | Zeit (h) | Edukt (g/l) | Produkt (g/l) |
| 5 | 14,5 | 0.5 | 4.5 |
| 10 | 14,5 | 1.9 | 8.0 |
| 20 | 14,5 | 6.7 | 10.2 |

### Beispiel 5: Einfluß der Eduktkonzentration

Ein Ansatz mit 25 g/l Hefe (Fermipan, Trockenhefe der Firma Gist-Brocades) und 60 g/l Glucose wurde mit verschiedenen Eduktkonzentrationen (siehe Schema I, Verbindung 1) bei 130 Upm und 31 °C getestet (siehe Tabelle V). Die Reaktion wurde in 250 ml Erlenmeyerkolben mit 50 ml Medium durchgeführt.

**Tabelle V:**

| Einfluß der Eduktkonzentration (25 g/l Fermipan) | | | |
|---|---|---|---|
| Keton vorgelegt (g/l) | Zeit (h) | Edukt (g/l) | Produkt (g/l) |
| 10 | 15 | 1.2 | 8.3 |
| 20 | 15 | 5.0 | 12.8 |
| 10 | 24 | 0.6 | 8.9 |
| 20 | 24 | 3.1 | 14.1 |
| 10 | 39 | 0.3 | 9.3 |
| 20 | 39 | 2.3 | 15.2 |
| 10(*) | 87 | 0.5 | 15.8 |

| | | | |
|---|---|---|---|
| (*) nochmal 5 g/l Keton zugegeben nach 63 h | | | |

### Beispiel 6: Einfluß der Produktkonzentration

In Gegenwart von 25 g/l Ferminpan-Trockenhefe, 10 g/l Edukt und 60 g/l Glucose wird die Reaktion mit verschiedenen vorgelegten Produktkonzentrationen bei 130 Upm und 31 °C (250 ml Erlenmeyerkolben mit 50 ml Medium) durchgeführt. Nach 15 h wurden nochmals 50 g/l Glucose nachgegeben. Nach den in Tabelle VI angegebenen Zeiten wurden Proben gezogen. Die vorgelegten Produktmengen hatten keinen Einfluß auf die Umsetzung in der Reaktion.

**Tabelle VI:**

| Einfluß der Produktkonzentration | | | | | |
|---|---|---|---|---|---|
| Bedingung: Produkt vorgelegt (g/l) | Laufzeit (h) | Edukt (g/l) | Produkt (g/l) | Umsatz (%) | Glucose (g/l) |
| 10 | 15 | 0.77 | 20.83 | 92.26 | 0.6 |
| 20 | 15 | 0.88 | 30.86 | 91.21 | 3.1 |
| 30 | 15 | 1.16 | 39.58 | 88.40 | 4.,3 |
| 40 | 15 | 1.34 | 49.15 | 86.62 | 52.2 |
| 10 | 40 | 0.36 | 19.89 | 92.26 | 0.3 |
| 20 | 40 | 0.50 | 29.75 | 91.21 | 0.3 |
| 30 | 40 | 0.64 | 41.02 | 88.40 | 28.2 |
| 40 | 40 | 0.63 | 49.54 | 86.62 | 44.1 |

### Beispiel 6: Einfluß der Produktkonzentration

In Gegenwart von 25 g/l Ferminpan-Trockenhefe und 60 g/l Glucose wurde die Reaktion mit verschiedenen vorgelegten Produktkonzentrationen ohne Eduktzugabe bei 130 Upm und 31 °C (250 ml Erlenmeyerkolben mit 50 ml Medium) durchgeführt. Nach 20 h wurden nochmals 50 g/l Glucose nachgegeben. Nach 48 h wurden Proben gezogen, in denen die vorgelegte Produktmenge in der GC-Analytik vollständig wiedergefunden wurde, daß heißt das Produkt wurde nicht verstoffwechselt.

### Beispiel 7: Einfluß der Temperatur

25 g/l Fermipan-Trockenhefe wurden in einem Ansatz mit 10 g/l Edukt bei 130 Upm und 31 °C (250 ml Erlenmeyerkolben mit 50 ml Medium) für 14,5 Stunden inkubiert. Es zeigte sich, daß die Reatkion in einem weiten Temperaturbereich durchführbar ist, mit einem leichten Maximum bei ca. 32 °C.

**Tabelle VII.**

| Einfluß der Temperatur | | | |
|---|---|---|---|
| Temperatur (°C) | Edukt g/l | Produkt g/l | Umsatz (%) |
| 24 | 2.1 | 9.1 | 76.3 |
| 28,5 | 1.1 | 9.6 | 86.8 |
| 30 | 1.0 | 9.3 | 87.1 |
| 32 | 0.6 | 9.0 | 91.1 |
| 37 | 0.6 | 8.5 | 90.9 |

### Beispiel 8: Einfluß der Hefekonzentration

Es wurde der Einfluß der Hefekonzentration auf die Reduktion getestet. Dazu wurden in einem Ansatz 50 g/l Glucose, 10 g/l Edukt (II) in Wasser vorgelegt und verschiedene Hefemengen (Fermipan-Trockenhefe) für die Reaktion eingesetzt. Die Ansätze wurden unter Schütteln (140 Upm) bei 30 °C inkubiert. Nach den in Tabelle VIII angegebenen Zeiten wurden Proben gezogen und analysiert. Mit zunehmender Hefekonzentration kann eine vorgegebene Eduktmenge rascher umgesetzt werden. Eine Sättigung konnte im getesteten Bereich nicht gefunden werden.

**Tabelle VIII:**

| Einfluß der Hefekonzentration auf die Reduktion | | | | | |
|---|---|---|---|---|---|
| Hefekonzentration (g/l) | Zeit (h) | Edukt (g/l) | Produkt (g/l) | Umsatz (%) | Glucose (g/l) |
| 12.5 | 3 | 8.2 | 1.2 | 13.2 | 41,5 |
| 25 | 3 | 6.8 | 2.5 | 26.8 | 20,3 |
| 50 | 3 | 4.9 | 5.1 | 50.7 | 0 |
| 100 | 3 | 3.6 | 7.2 | 66.9 | 0 |
| 12.5 | 5.5 | 7.5 | 2.1 | 21.7 | 32,0 |
| 25 | 5.5 | 5.3 | 4.6 | 46.3 | 0 |
| 50 | 5.5 | 3.0 | 6.4 | 67.8 | 20,2 |
| 100 | 5.5 | 1.7 | 9.4 | 84.6 | 8,9 |
| 12.5 | 8 | 6.9 | 2.9 | 29.7 | 17,7 |
| 25 | 8 | 3.5 | 5.8 | 62.2 | 36,1 |
| 50 | 8 | 2.0 | 8.3 | 80.7 | 0, |
| 100 | 8 | 0.9 | 10.1 | 91.5 | 0 |
| 12.5 | 13 | 5.0 | 5.6 | 52.8 | 0 |
| 25 | 13 | 1.4 | 8.7 | 86.0 | 0 |
| 50 | 13 | 0.9 | 10.6 | 92.3 | 0 |
| 100 | 13 | 0.5 | 11.7 | 95.6 | 0 |
| 12.5 | 25 | 3.0 | 8.9 | 74.9 | 0 |
| 25 | 25 | 0.9 | 10.7 | 92.6 | 0 |
| 50 | 25 | 0.5 | 11.8 | 95.7 | 0 |
| 100 | 25 | 0.5 | 12.7 | 96.4 | 0 |

### Beispiel 9: Ethanol als C-Quelle

Verschiedene in Tabelle IX angegebene Ethanolkonzentrationen wurden als Kohlenstoffquelle für die Reduktion getestet. Dazu wurden in einem Ansatz 25 g/l Trockenhefe (Fermipan), 5 g/l Edukt (II) in Wasser vorgelegt und verschiedene Ethanolkonzentrationen für die Reaktion eingesetzt. Die Ansätze wurden unter Schütteln (130 Upm) bei 30 °C inkubiert. Nach den in Tabelle IX angegebenen Zeiten wurden Proben gezogen und analysiert. Es zeigte sich, daß Ethanol als Kohlenstoffquelle ebenfalls für die Reduktion geeignet ist. Jedoch ist die Reaktion deutlich langsamener.

**Tabelle IX:**

| Ethanol als Kohlenstoffquelle | | | | | |
|---|---|---|---|---|---|
| Ethanol vorgelegt (g/l) | Zeit (h) | Ethanol (g/l) | Edukt (g/l) | Produkt (g/l) | Umsatz (%) |
| 26 | 8 | 25,9 | 4.1 | 1.1 | 21.8 |
| 60 | 8 | 59,2 | 4.4 | 1.2 | 22.0 |
| 142 | 8 | 144,5 | 4.3 | 0.5 | 10.9 |
| 26 | 15 | 18,9 | 3.1 | 2.2 | 41.2 |
| 60 | 15 | 56,3 | 3.8 | 1.7 | 31.3 |
| 142 | 15 | 110,5 | 4.0 | 0.7 | 14.1 |

## Patentansprüche

1. Verfahren zur Herstellung von enantiomerenreinen Alkoholen der Formel I (Ia oder Ib) 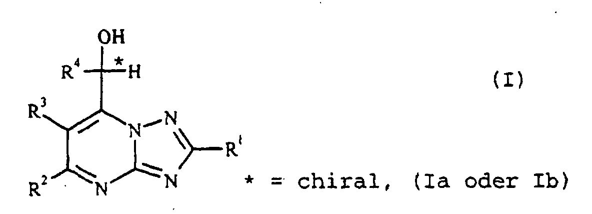 in der die Substituenten folgende Bedeutung haben:
R¹
Wasserstoff oder substituiertes oder unsubstituiertes C₁-C₆-Alkyl-, C₁-C₆-Alkoxy- oder C₁-C₆-Alkanoyl-,
R² und R³
unabhängig voneinander Wasserstoff oder substituiertes oder unsubstituiertes C₁-C₆-Alkyl-, C₁-C₆-Alkoxy-, C₁-C₆-Alkanoyl-, C₁-C₆-Alkylthio-, C₁-C₆-Alkylsulphinyl- oder C₂-C₆-Alkylsulphonyl-,
R4
R⁴ substituiertes oder unsubstituiertes C₁-C₆-Alkyl- , C₃-C₈-Cycloalkyl-,
**dadurch gekennzeichnet, daß** man Verbindungen der Formel II, in der die Substituenten R¹ bis R⁴ die oben genannten Bedeutungen haben, in wäßriger Lösung 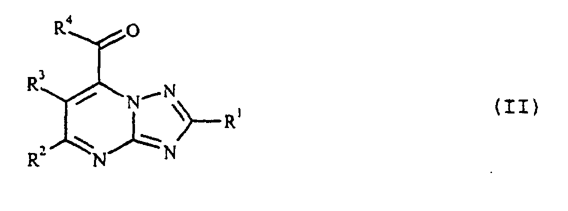 in Gegenwart einer Kohlenstoffquelle und eines Mikroorganismus oder eines Reduktionsmittels, eines Cofaktors und eines Enzyms zu Verbindungen der Formel I reduziert.

2. Verfahren nach Anspruch 1, **dadurch gekennzeichnet, daß** man die Reaktion in Gegenwart eines selektiv reduzierenden Mikroorganismus oder eines selektiv reduzierenden Enzyms durchführt.

3. Verfahren nach Anspruch 1 oder 2, **dadurch gekennzeichnet, daß** man die Reaktion in Gegenwart eines Mikroorganismus ausgewählt aus der Gruppe Hefen, Pilze oder Bakterien oder einer Alkoholdehydrogenase durchführt.

4. Verfahren nach einem der Ansprüche 1 bis 3, **dadurch gekennzeichnet, daß** man die Reaktion in Gegenwart eines Mikroorganismuses ausgewählt aus der Gruppe der Gattungen Alcaligenes, Aspergillus, Beauveria, Candida, Cryptococcus, Curvularia, Diplodia, Endomycopsis, Geotrichum, Hansenula, Kloeckera, Kluveromyces, Lactobacillus, Mucor, Nocardia, Penicillium, Pfaffia, Pichia, Pseudomonas, Rhodococcus, Rhodotorula, Saccharomyces, Schizosaccharomyces, Sporidiobolus, Streptomyces, Torulopsis, Yarrowia durchführt.

5. Verfahren nach einem der Ansprüche 1 bis 4, **dadurch gekennzeichnet, daß** man die Reaktion in Gegenwart der Gattung Saccharomyces durchführt.

6. Verfahren nach einem der Ansprüche 1 bis 5, **dadurch gekennzeichnet, daß** man die Reaktion in Wasser als einzigem Lösungsmittel oder in einem Wasser/organischen Lösungsmittelgemisch durchführt.

7. Verfahren nach einem der Ansprüche 1 bis 6, **dadurch gekennzeichnet, daß** man die Reaktion in einem Temperaturbereich von 0 °C bis 50 °C durchführt.

## Claims

1. A process for preparing enantiomerically pure alcohols of the formula I (Ia or Ib) 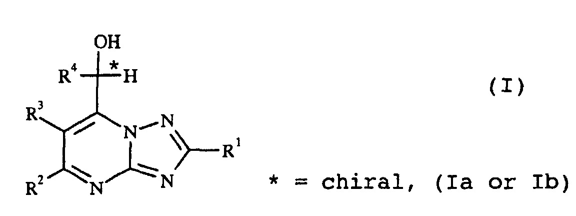 where the substituents have the following meanings:
R¹
hydrogen or substituted or unsubstituted C₁-C₆-alkyl, C₁-C₆-alkoxy or C₁-C₆-alkanoyl,
R² and R³
independently of one another hydrogen or substituted or unsubstituted C₁-C₆-alkyl, C₁-C₆-alkoxy, C₁-C₆-alkanoyl, C₁-C₆-alkylthio, C₁-C₆-alkylsulfinyl or C₁-C₆-alkylsulfonyl,
R4
substituted or unsubstituted C₁-C₆-alkyl, C₃-C₈-cycloalkyl,
which comprises reducing compounds of the formula II where the substituents R¹ to R⁴ have the abovementioned meanings 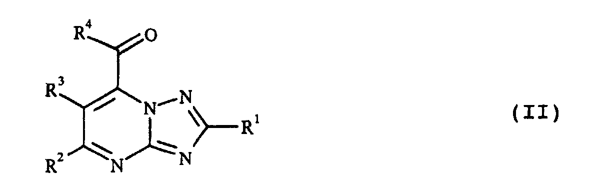 in aqueous solution in the presence of a carbon source and of a microorganism or of a reducing agent, of a cofactor and of an enzyme, to compounds of the formula I.

2. A process as claimed in claim 1, wherein the reaction is carried out in the presence of a selectively reducing microorganism or of a selectively reducing enzyme.

3. A process as claimed in claim 1 or 2, wherein the reaction is carried out in the presence of a microorganism selected from the group of yeasts, fungi or bacteria or of an alcohol dehydrogenase.

4. A process as claimed in any of claims 1 to 3, wherein the reaction is carried out in the presence of a microorganism selected from the group of genera Alcaligenes, Aspergillus, Beauveria, Candida, Cryptococcus, Curvularia, Diplodia, Endomycopsis, Geotrichum, Hansenula, Kloeckera, Kluyveromyces, Lactobacillus, Mucor, Nocardia, Penicillium, Pfaffia, Pichia, Pseudomonas, Rhodococcus, Rhodotorula, Saccharomyces, Schizosaccharomyces, Sporidiobolus, Streptomyces, Torulopsis, Yarrowia.

5. A process as claimed in any of claims 1 to 4, wherein the reaction is carried out in the presence of the genus Saccharomyces.

6. A process as claimed in any of claims 1 to 5, wherein the reaction is carried out in water as sole solvent or in a water/organic solvent mixture.

7. A process as claimed in any of claims 1 to 6, wherein the reaction is carried out at a temperature in the range from 0 °C to 50 °C.

## Revendications

1. Procédé pour la préparation d'alcools purs quant aux énantiomères de formule I (Ia ou Ib) 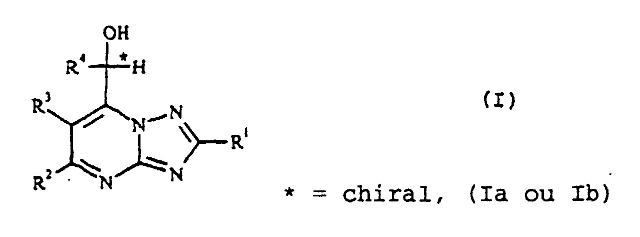 dans laquelle les substituants ont la signification suivante:
R¹
hydrogène ou alkyle en C₁-C₆, alcoxy en C₁-C₆ ou alcanoyle en C₁-C₆, substitué ou non substitué,
R² et R³
indépendamment l'un de l'autre, hydrogène ou alkyle en C₁-C₆, alcoxy en C₁-C₆, alcanoyle en C₁-C₆, alkylthio en C₁-C₆, alkylsulphinyle en C₁-C₆ ou alkylsulfonyle en C₁-C₆, substitué ou non substitué,
R4
alkyle en C₁-C₆, cycloalkyle en C₃-C₈, substitué ou non substitué,
**caractérisé par le fait qu'**on réduit des composés de formule II dans laquelle les substituants R¹ à R⁴ ont les significations susdites, en solution aqueuse 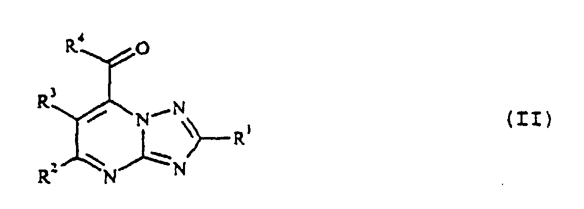 en présence d'une source de carbone et d'un micro-organisme ou d'un agent réducteur, d'un cofacteur ou d'une enzyme pour former des composés de formule I.

2. Procédé selon la revendication 1, **caractérisé par le fait qu'**on effectue la réaction en présence d'un micro-organisme sélectivement réducteur ou d'une enzyme sélectivement réductrice.

3. Procédé selon la revendication 1 ou 2, **caractérisé par le fait qu'**on effectue la réaction en présence d'un micro-organisme choisi dans le groupe des levures, des champignons ou des bactéries ou d'une alcool déshydrogénase.

4. Procédé selon l'une des revendications 1 à 3, **caractérisé par le fait qu'**on effectue la réaction en présence d'un micro-organisme choisi dans le groupe des espèces Alcaligenes, Aspergillus, Beauveria, Candida, Cryptococcus, Curvularia, Diplodia, Endomycopsis, Geotrichum, Hansenula, Kloeckera, Kluveromyces, Lactobacillus, Mucor, Nocardia, Penicillium, Pfaffia, Pichia, Pseudomonas, Rhodococcus, Rhodotorula, Saccharomyces, Schizosaccharomyces Sporidiobolus, Streptomyces, Torulopsis, Yarrowia.

5. Procédé selon l'une des revendications 1 à 4, **caractérisé par le fait qu'**on effectue la réaction en présence de l'espèce Saccharomyces.

6. Procédé selon l'une des revendications 1 à 5, **caractérisé par le fait qu'**on effectue la réaction dans l'eau en tant que solvant unique ou dans un mélange d'eau/solvant organique.

7. Procédé selon l'une des revendications 1 à 6, **caractérisé par le fait qu'**on effectue la réaction dans un intervalle de température de 0°C à 50°C.
